# FASCICULE DE BREVET EUROPEEN

(11) **EP 3 269 426 B1**
(45) Date de publication et mention de la délivrance du brevet: **22.04.2020**
(21) Numéro de dépôt: 17178224.6
(22) Date de dépôt: 27.06.2017
(51) Int. Cl.: A61K 8/9789, A61Q 19/00, A61Q 19/08

(54) **EXTRAIT D'AESCULUS HIPPOCASTANUM**
ROSSKASTANIENEXTRAKT
EXTRACT OF AESCULUS HIPPOCASTANUM

(30) Priorité: 13.07.2016 FR 1656736
(43) Date de publication de la demande: 17.01.2018
(73) Titulaire: GATTEFOSSE SAS, 69800 Saint Priest (FR)
(72) Inventeur: CHARTON, Virginie, 69740 GENAS (FR); CAPRIN, Benoit, 69008 LYON (FR); BECHETOILLE, Nicolas, 69530 BRIGNAIS (FR)
(74) Mandataire: Cabinet Laurent & Charras

(56) Documents cités:
- WO-A2-2009/107853
- DE-B- 1 194 529
- FR-A- 1 422 009
- US-A1- 2005 048 008
- DATABASE GNPD [Online] MINTEL; 31 mai 2016 (2016-05-31), "Protect Barrier Rich", XP002764077, Database accession no. 3967717

## Description

L'invention concerne un extrait de marronnier d'Inde ou *Aesculus hippocastanum.* Elle vise également une composition cosmétique comprenant ledit extrait. Elle se rapporte enfin à l'utilisation de l'extrait pour le traitement cosmétique de la peau et/ou des muqueuses.

Le marronnier d'Inde *(Aesculus hippocastanum)* est un grand arbre pouvant atteindre 30 m de hauteur, sa croissance est rapide, son tronc robuste et droit, et son port en boule. L'écorce brun rougeâtre reste longtemps lisse, puis se fissure dans le sens de la longueur et s'écaille et se détache par plaques. Son feuillage est caduc. Les bourgeons pointus apparaissent en automne et sont protégés par une résine très collante. Les feuilles sont opposées et palmées, grandes (30 à 50 cm). L'inflorescence est constituée de fleurs zygomorphes blanches souvent tachetées de rouge ou de jaune. Le fruit est une capsule verte (bogue) à paroi épaisse et lisse qui renferme une (parfois 2 ou 3) grosse graine brune, lisse et luisante appelée « marron ». Cette graine n'est pas comestible.

Le marronnier d'Inde est une espèce végétale particulièrement exploitée en cosmétique, dermocosmétique et dans l'industrie pharmaceutique.

On connaît l'utilisation d'extraits de graine de marronnier d'Inde, contenant principalement un mélange de saponines triterpéniques appelé aescine (ou escine), dans de nombreuses préparations pour traiter des insuffisances veineuses chroniques et pour soulager certains symptômes tels que les jambes lourdes et gonflées. L'aescine réduit la fragilité capillaire en inhibant l'enzyme hyaluronidase. L'aescine est aussi anti-inflammatoire, anti-tumorale, neuroprotective, antioxydante. Si de nombreuses préparations cosmétiques incluent des extraits de graine d'*Aesculus hippocastanum* pour ses effets sur la circulation sanguine, d'autres effets bénéfiques sur la peau lui sont attribués. Un extrait de graine d'*Aesculus hippocastanum* présente des propriétés antioxydantes supérieures à celles de l'acide ascorbique et protège les cellules contre le stress oxydatif. Un extrait de graine d'*Aesculus hippocastanum* entraine la contraction des fibroblastes et diminue les rides des contours des yeux. Le document WO2009/107853 A2 décrit par exemple un agent comprenant une saponine triterpénique obtenue par extraction éthanol-eau des graines d'*Aesculus hippocastanum,* qui stimule la contraction des fibroblastes dermiques pour prévenir et améliorer les rides et le relâchement de la peau au cours du vieillissement. Les extraits de graines d'*Aesculus hippocastanum* améliorent les performances protectrices des filtres solaires. Le document WO2009/107853 A2 décrit un extrait de graine d'*Aesculus hippocastanum* riche en saponine pour un effet anti âge. Cet extrait est obtenu au moyen d'un solvant alcoolique.

On connaît aussi l'utilisation d'extraits d'écorce de marronnier d'Inde. Son écorce aux propriétés astringentes est employée contre les diarrhées par voie orale et comme antiseptique en application locale sur les plaies et les ulcères. Les extraits d'écorce contiennent principalement des coumarines telles que l'esculine (ou aesculine, ou esculoside), la fraxine, l'esculétine et la fraxétine, et également des tanins, considérés comme vasculoprotecteurs et utilisés notamment pour le traitement des hémorroïdes, des œdèmes.

Le document US 2005/048008 A1 décrit des compositions cosmétiques pour traiter le vieillissement cutané et pour améliorer l'aspect de la peau. Ce document décrit un extrait d'*Aesculus hippocastanum* sans pour autant préciser à partir de quelle partie de la plante l'extrait est obtenu. En outre, rien n'est indiqué concernant la nature du solvant utilisé.

L'utilisation d'extrait de fleur d'*Aesculus hippocastanum* est décrite en médecine traditionnelle à la fois pour un usage interne ou externe, pour traiter l'inflammation veineuse, les varices légères, les hémorroïdes et les engelures. La fleur renferme des flavonoïdes et principalement des dérivés glycosylés du kaempférol et de la quercétine. La présence de dérivés de types tanins, coumarines, amines et acides aminés est également rapportée. Il existe aussi une monographie inscrite à la Pharmacopée française pour une préparation homéopathique à partir des fleurs d'*Aesculus hippocastanum.* Il s'agit dans ce cas d'une teinture mère obtenue par extraction hydro-éthanolique (45/55 V/V) de fleur fraîche réalisée selon la technique générale de préparation des teintures mères. Le document MINTEL (database accession number 3967717) décrit une composition cosmétique pour le traitement de la peau comprenant un extrait de fleur d'*Aesculus hippocastanum.* Ce document ne décrit pas la nature du solvant utilisé pour obtenir l'extrait de fleurs.

Pour une gamme de polarité de solvants comprise entre celle de l'éthanol et de l'eau, la présence des saponosides et/ou de coumarines peut induire une potentielle toxicité des extraits, nonobstant leur efficacité décrite précédemment.

D'autre part, le choix à effectuer parmi les solvants disponibles pour l'obtention des extraits pose un certain nombre de difficultés.

Les solvants aqueux nécessitent l'ajout d'un conservateur microbiologique pour garantir leur stabilité. Or le champ d'utilisation des conservateurs est de plus en plus restreint en raison notamment des données toxicologiques requises. Le propylène glycol s'est révélé potentiellement toxique, et tend actuellement à être évité par les industriels. Le butylène glycol agrosourcé est relativement coûteux, ce qui limite son utilisation industrielle. Le propanediol agrosourcé présente l'inconvénient d'être issu de maïs génétiquement modifiés (OGM), qui ne sont pas facilement acceptés par le consommateur et au sujet desquels il peut être prudent de garder des réserves. L'éthanol, bien que présentant un bon pouvoir d'extraction seul ou en mélange dans l'eau, est particulièrement volatil et inflammable, ce qui engendre des problématiques de transport et de stockage. D'autre part, en raison de son caractère desséchant, ce solvant peut perturber le film hydrolipidique de la peau et provoquer des irritations pour les peaux sensibles.

Par ailleurs, les extraits de plantes étant souvent destinés à être formulés dans des compositions cosmétiques, il importe d'éviter les solvants d'extraction comprenant des sels ou des acides, dont on retrouvera nécessairement au moins des traces dans l'extrait. En effet, il est bien connu que la formulation, en gel ou en émulsion, d'ingrédients à base de sels au-delà d'une dose de 0,1% est particulièrement complexe. En outre, les acides, en abaissant le pH des extraits obtenus, ont pour effet de rendre la formulation en gel ou en émulsion difficile. En particulier, les produits cosmétiques destinés à l'application cutanée doivent avoir un pH adapté, de préférence proche de celui de la peau (environ pH 6,5), ou neutre. Dans ces conditions, l'utilisation de solvants contenant des acides requiert par conséquent l'ajout d'excipients, notamment des régulateurs de pH.

En d'autres termes, le problème que se propose de résoudre l'invention est celui de mettre au point un extrait d'*Aesculus hippocastanum* qui ne présente pas les inconvénients ci-dessus exposés, en particulier qui soit dépourvu de coumarine, notamment d'aesculine, et dépourvu de saponoside, en particulier d'aescine.

Un autre problème que se propose de résoudre l'invention est celui de mettre au point un extrait d'*Aesculus hippocastanum* qui soit dépourvu d'effets toxiques ou irritants.

Parallèlement, on recherche en permanence des solutions pour améliorer l'aspect de la peau, ou encore prévenir ou diminuer les rides ou plus généralement le relâchement cutané. Une des voies envisagées est celle de stimuler l'expression du collagène IV, du collagène VII et de la fibronectine, ces protéines étant toutes impliquées dans la jonction dermo-épidermique (JDE).

La JDE est impliquée dans plusieurs processus biologiques comme la réparation tissulaire, l'attachement, la migration, la prolifération et la différenciation épidermique. La JDE est également un lieu de stockage des cytokines, un réservoir de facteurs de croissance, un filtre sélectif pour contrôler les échanges de cellules, macromolécules et nutriments et une barrière chimique et physique. Cette dernière fonction est particulièrement importante car c'est par la JDE que la cohésion entre l'épiderme et le derme s'effectue, ce qui permet une résistance aux forces de traction cutanées externes.

La JDE est composée des protéines des hémisdesmosomes et d'une membrane basale épidermique qui est un réseau moléculaire complexe et organisé. La membrane basale épidermique est formée de trois couches superposées distinctes :
- la *lamina lucida*: fins filaments d'ancrage qui s'insèrent dans la *lamina densa*;
- la *lamina densa* : fin feuillet de matrice extracellulaire hautement spécialisé résultant de l'agencement des réseaux protéiques de collagène de type IV et de laminine, associés à la fibronectine;
- la *lamina reticularis*: zone fibrillaire qui est constituée de fibrilles d'ancrage dont le composant principal est le collagène de type VII.

Le processus de vieillissement conduit à des modifications importantes dans la structure et la composition de la JDE. En particulier, la dégradation protéolytique des constituants de la membrane basale perturbe l'organisation structurale de la JDE, qui au cours du vieillissement perd ses propriétés viscoélastiques. La composition de la JDE semble également beaucoup varier en fonction de l'âge, le collagène IV ainsi que le collagène VII étant réduits.

Par conséquent, un autre problème que se propose de résoudre l'invention est celui de mettre au point une composition cosmétique qui présente des propriétés intéressantes de stimulation de la synthèse des protéines de la JDE, en particulier, collagène IV, collagène VII et fibronectine.

Le Demandeur a résolu l'ensemble de ces problèmes en mettant au point un extrait d'*Aesculus Hippocastanum* susceptible d'être obtenu par un procédé d'extraction comprenant une étape d'extraction solide/liquide d'au moins une partie de la plante, suivie d'une seconde étape de séparation solide /liquide et enfin d'une troisième étape de récupération de la phase liquide.

L'invention se caractérise en ce que le solvant consiste en un mélange de fructose et de glycérine comprenant éventuellement de l'eau.

L'extrait de l'invention est dépourvu de saponoside et de coumarine. Il est composé majoritairement de flavonoïdes, de dérivés catéchiques et d'acides aminés.

Avantageusement, l'extrait présente une teneur en flavonoïdes comprise entre 10 et 200 mg/100g d'extrait, avantageusement entre 50, de préférence 75 et 100 mg/100g d'extrait.

De même, l'extrait présente de préférence une teneur en acides aminés libres comprise entre 10 et 200 mg/100g d'extrait, avantageusement entre 50, de préférence 75 et 100 mg/100g d'extrait.

L'extrait de l'invention est donc particulièrement intéressant car dépourvu de composants susceptibles d'irriter la peau ou les muqueuses. Les compositions cosmétiques le comprenant sont donc aussi moins susceptibles de créer des réactions cutanées.

D'autre part, le solvant mis au point par le Demandeur est exempt d'acide organique, de composés inorganiques et de sels. De surcroît ce solvant est dépourvu de glycol, d'alcool, de sels minéraux, et d'acides. Ainsi, sa composition chimique n'entrave pas la formulation en gel ou émulsion.

En conséquence, l'extrait obtenu avec ce solvant, et qui comprend nécessairement des traces du solvant d'extraction, possède les mêmes avantages et est particulièrement adapté à la formulation de produits cosmétiques.

Selon une première caractéristique, le solvant peut contenir de l'eau, ou en être dépourvu. La présence d'eau dans le solvant a toutefois pour avantage de fluidifier le solvant, et ainsi de faciliter l'extraction. En d'autres termes et selon un premier mode de réalisation avantageux, le solvant consiste en un mélange de fructose, de glycérine et d'eau.

En outre, lorsque les composants de ce solvant sont présents dans certains rapports molaires, le solvant possède les propriétés d'un solvant NaDES, c'est-à-dire un solvant eutectique composé de molécules retrouvées dans la nature, et liées entre elles par des interactions intermoléculaires, en particulier des liaisons hydrogènes. Le solvant est notamment liquide à température ambiante, ce qui facilite la mise en œuvre du procédé conduisant à l'extrait de l'invention.

Dans un mode de réalisation particulier, le solvant consiste en un mélange de fructose, de glycérine et d'eau dans des proportions molaires comprises entre 1:1:3 et 1:1:7, préférentiellement dans des proportions molaires d'environ 1:1:5.

En pratique, le solvant utilisé peut être produit en mélangeant le fructose et la glycérine, ou le fructose, la glycérine et l'eau, dans un réacteur agité, jusqu'à l'obtention d'un mélange limpide incolore. Ce mélange peut être réalisé à une température comprise entre 2°C et 100°C et pendant 0,5 à 6 heures, préférentiellement 40°C à 70°C pendant 1 à 2 heures.

Selon l'invention, toute ou partie de la plante *Aesculus Hippocastanum* peut être mise en œuvre dans le procédé de l'invention. En pratique, la partie de la plante mise en œuvre dans le procédé est choisie dans le groupe comprenant la fleur, la graine, la feuille.

De manière particulièrement préférée, la partie de la plante mise en œuvre est la fleur. Il peut s'agir d'une fleur fraiche, congelée, sèche, entière, coupée ou broyée. Avantageusement, il s'agit d'une fleur sèche et broyée.

L'extrait selon l'invention est susceptible d'être obtenu par un procédé mettant en œuvre une étape d'extraction solide/liquide.

L'extraction solide/liquide peut être effectuée par différentes techniques bien connues de l'homme du métier, telles que macération, remacération, digestion, macération dynamique, décoction, extraction en lit fluide, extraction assistée par micro-ondes, extraction assistée par ultra-sons, extraction à contre-courant, percolation, re-percolation, lixiviation, extraction sous pression réduite, diacolation.

En pratique le rapport massique plante/solvant appliqué pour l'étape d'extraction est compris entre 1/99 et 10/90. L'étape d'extraction est effectuée de préférence à une température comprise entre 2 et 100°C, plus préférentiellement entre 20 et 80°C. L'étape d'extraction peut être maintenue pendant quelques minutes à plusieurs jours.

De manière à optimiser l'extraction des composés actifs tout en protégeant ces composés de l'oxydation par l'oxygène de l'air, l'étape d'extraction solide/liquide est avantageusement réalisée sous agitation et/ou sous atmosphère d'azote.

Selon l'invention, l'étape d'extraction solide/liquide est suivie d'une étape de séparation solide/liquide, l'objectif étant de récupérer la phase liquide, aussi appelée filtrat de séparation solide/liquide, contenant la matière active. Cette séparation peut être effectuée par toute technique connue de l'homme du métier, en particulier l'égouttage, le pressage, l'essorage, la centrifugation ou la filtration.

De manière optionnelle, l'étape de séparation liquide/solide peut être suivie d'une étape de concentration, laquelle permet d'obtenir un concentré, sous forme liquide ou semi-solide selon le facteur de concentration. En pratique, l'étape de concentration peut être effectuée par évaporation sous pression réduite ou osmose inverse.

De préférence, le filtrat de séparation solide/liquide ou le concentré subissent en outre une ou plusieurs étapes de clarification. Pour réaliser cette étape de clarification, l'homme du métier pourra utiliser tout type de filtration connue dans le domaine considéré.

Enfin, en vue d'un conditionnement, le procédé permettant l'obtention de l'extrait selon l'invention peut comprendre une filtration stérilisante. La filtration stérilisante est classiquement réalisée en filtrant le produit à travers un filtre comprenant des pores d'un diamètre d'environ 0,22 µm. De préférence, l'étape de filtration stérilisante est l'étape finale du procédé.

Le Demandeur a en outre découvert que l'extrait de l'invention possède des propriétés biologiques particulièrement intéressantes, en particulier sur la stimulation de la synthèse de protéines de la peau, lesquelles propriétés ne sont pas obtenues avec des extraits d'*Aesculus Hippocastanum* obtenus par la voie classique d'extraction hydroéthanolique.

En effet et tel que détaillé dans la partie expérimentale, l'extrait de l'invention a notamment pour propriété de stimuler l'expression du collagène IV, du collagène VII et de la fibronectine, ces protéines étant toutes impliquées dans la JDE.

L'extrait de l'invention, stimulant la synthèse du collagène IV, du collagène VII et de la fibronectine, est donc particulièrement intéressant pour des utilisations cosmétiques, en particulier visant l'amélioration de l'aspect de la peau, ou encore la prévention ou la diminution des rides et du relâchement cutané et plus généralement pour le traitement cosmétique de la peau et des muqueuses.

L'invention vise donc aussi l'utilisation, de préférence non thérapeutique, de l'extrait selon l'invention ou de compositions le comprenant, pour le traitement cosmétique de la peau et/ou des muqueuses, en particulier pour l'amélioration de l'aspect de la peau et/ou des muqueuses, pour l'amélioration de la résistance et/ou de l'élasticité cutanée, pour le traitement ou la prévention du vieillissement de la peau, des rides, ou du relâchement cutané.

Dans cette perspective, l'invention vise aussi des compositions comprenant l'extrait de l'invention, de préférence des compositions cosmétiques, c'est-à-dire adaptées à l'application topique sur la peau et/ou les muqueuses, et/ou les phanères.

De préférence, dans la composition de l'invention, l'extrait selon l'invention représente entre 0,1 % et 10 %, de préférence entre 0,5 % et 5 % en poids de la composition.

La composition selon l'invention peut se présenter sous toutes les formes galéniques normalement utilisées pour une application topique sur la peau et/ou les muqueuses, et/ou les phanères, par exemple sous forme anhydre, sous forme d'une émulsion huile-dans-eau, d'une émulsion eau-dans-huile, d'une émulsion multiple, d'une émulsion siliconée, d'une microémulsion, d'une nanoémulsion, d'un gel, d'une solution aqueuse ou d'une solution hydro-alcoolique.

Cette composition peut être plus ou moins fluide et se présenter sous forme d'une crème blanche ou colorée, d'une pommade, d'un lait, d'une lotion, d'un sérum, ou d'un gel.

La composition cosmétique et/ou dermatologique peut contenir les excipients habituellement utilisés dans les domaines cosmétique et dermatologique, tels que les matières grasses, les tensioactifs détergents et/ou conditionnants, les émulsionnants et co-émulsionnants, les gélifiants hydrophiles ou lipophiles, les conservateurs, les antioxydants, les solvants, les agents exfoliants, les parfums, les charges, les filtres hydrophiles et lipophiles, les matières colorantes, les neutralisants, les agents pro-pénétrants, et les polymères. Ces types d'excipients sont tous bien connus de l'homme du métier.

En pratique, les quantités de ces différents excipients sont celles classiquement utilisées dans les domaines considérés, et la somme des excipients représente de préférence 0,01% à 30% du poids total de la composition.

Comme matières grasses appropriées, on peut citer les huiles minérales, les huiles d'origine animale (telle la lanoline), les huiles végétales, les huiles de synthèse (comme par exemple l'isopropyl le myristate, l'octyldodecyl, l'isostearyl isostearate, le decyl oleate, l'isopropyl le palmitate), les huiles siliconées (la cyclomethicone, la dimethicone) et les huiles fluorées. On peut utiliser comme matières grasses des alcools gras, des acides gras, des cires et des gommes, et en particulier des élastomères de silicone.

Comme tensioactifs détergents et/ou conditionnants appropriés, on peut citer les tensioactifs non ioniques, anioniques, cationiques ou amphotères, et leurs mélanges, tels que par exemple les alkylsulfates, les alkyléthersulfates tels que le lauryl éther sulfate de sodium, les alkyl bétaïnes telles que la cocamidopropylbetaïne, ou les sels d'ammonium quaternaires.

Comme émulsionnants et co-émulsionnants appropriés, on peut citer par exemple les esters de polyglycérols et d'acide gras, les esters de sucrose et d'acide gras, les esters de sorbitane et d'acide gras, les esters d'acide gras et de sorbitane oxyéthylénés, les ethers d'alcool gras et de PEG, les esters de glycérol et d'acide gras, les alkyl sulfates, les alkyl ether sulfates, les alkyl phosphates, les alkyl polyglucosides, les dimethicone copolyols.

Comme gélifiants hydrophiles appropriés, on peut citer par exemple les polymères carboxyvinyliques (carbomers), les copolymères acryliques tels que les copolymères d'acrylates/alkylacrylates, les polyacrylamides, les polysaccharides tels que la gomme xanthane, la gomme guar, les gommes naturelles telles que la gomme de cellulose et dérivés, les amidons et leurs dérivés, les argiles et les copolymères d'acide 2-acrylamido-2-méthylpropane.

Comme gélifiants lipophiles appropriés, on peut citer par exemple les argiles modifiées comme les bentones, les sels métalliques d'acides gras, la silice hydrophobe et l'éthylcellulose.

Comme conservateurs appropriés, on peut citer par exemple les acides benzoïque, sorbique, propionique, salicylique, dehydroacétique et leurs sels, l'alcool benzylique, l'éthylhexylglycérine, les parabens, leurs sels et esters, le triclosan, l'imidazolidinyl urée, le 5 phenoxyethanol, la DMDM hydantoïne, le diazolidinyl urée, la chlorphenesine.

Comme antioxydants appropriés, on peut citer par exemple les agents chelatants tels que l'EDTA et ses sels, le metabisulfite de sodium, les acides salicylique, ascorbique et citrique et leurs sels, le tartrate de sodium, le gluconate de sodium, les caroténoïdes et les tocophérols.

Comme solvants utilisables dans la composition cosmétique (distinct du solvant d'extraction), on peut citer l'eau, l'éthanol, la glycérine, le propylène glycol, le propanediol, le butylène glycol, le sorbitol.

Comme agents exfoliants appropriés, on peut citer par exemple les exfoliants chimiques tels que les AHA, et les exfoliants physiques tels que les poudres naturelles ou synthétiques.

Comme charges appropriées, on peut citer par exemple le talc, le kaolin, le mica, la serecite, le magnesium carbonate, l'aluminium silicate, le magnesium silicate, les poudres organiques telles que le nylon.

Comme filtres appropriés, on peut citer par exemple les filtres UVA et UVB classiquement utilisés tels que la benzophenone-3, le butyl methoxydibenzoyl methane, l'octocrylène, l'octyl methoxycinnamate, le 4-methylbenzylidene camphor, l'octyl salycylate, le tacephthalydene dicamphor sulfanic acid, le drométrizole trisiloxane, le methylene bis benzotriazolyl tetramethylbutylphenol, le bis-ethylhexyloxyphenol methoxyphenyl triazine, l'ethylhexyl triazone, et le Diethylamino Hydroxybenzoyl Hexyl Benzoate. On citera également les filtres physiques TiO2 et ZnO sous leurs formes micrométriques et nanométriques, enrobés ou non enrobés.

Comme colorants appropriés, on peut citer par exemple les colorants lipophiles, les colorants hydrophiles, les pigments et les nacres habituellement utilisés dans les compositions cosmétiques ou dermatologiques, et leurs mélanges.

Comme neutralisants appropriés, on peut citer par exemple la soude, la triethanolamine, l'aminomethyl propanol, l'hydroxyde de potassium.

Comme agents pro-pénétrants appropriés, on peut citer par exemple les alcools et glycols (éthanol, propylène glycol), l'éthoxydiglycol, les alcools et acides gras (acide oléique), les esters d'acides gras, le dimethyl isosorbide.

La composition de l'invention peut également contenir en outre d'autres actifs que l'extrait selon l'invention. Comme actifs appropriés, on peut citer par exemple les anti-radicalaires ou plus généralement les antioxydants, les blanchissants, les pigmentants, les émollients, les hydratants, les anti-séborrhéiques, les anti-inflammatoires, les anti-acnéiques, les agents kératolytiques et/ou desquamants, les agents anti-rides et tenseurs, les agents drainants, les agents anti-irritants, les agents apaisants, les vitamines et leurs mélanges, les agents matifiants, les actifs anti-âge tel que le rétinol, les cicatrisants, les antiseptiques et les huiles essentielles.

L'invention et les avantages qui en découlent ressortiront bien des exemples de réalisation suivants.

### EXEMPLES

### Exemple 1: Fabrication des extraits d'Aesculus hippocastanum

On mélange 1 partie de fleurs séchées et broyées ou de graines broyées d'*Aesculus hippocastanum* et 19 parties d'un mélange éthanol/eau 70:30 poids/poids (noté solvant EtOH/Eau) ou d'un mélange Fructose/Glycérine/Eau en ratio molaire 1:1:5 (noté solvant FGE). Le mélange est extrait à 70°C pendant 3h puis on filtre l'extrait. On obtient ainsi un extrait de fleur hydro-éthanolique limpide de couleur marron jaune (noté extrait fleur EtOH/eau) et un extrait de fleur Fructose/Glycérine/Eau (noté extrait fleur FGE) limpide de couleur ambrée. Enfin, on obtient un extrait de graine hydro-éthanolique (noté extrait graine EtOH/eau) et un extrait de graine Fructose/Glycérine/Eau (noté extrait graine FGE) limpides et de couleur jaune clair.

### Exemple 2: Caractérisation de la composition en en acides aminés libres dans les extraits d'Aesculus hippocastanum

Les teneurs en acides aminés libres contenus dans les extraits obtenus à l'exemple 1 sont quantifiés par HPLC (Shimadzu R_LCS_01) sur une colonne Waters Novapack C18. L'extrait FGE est dérivé après dilution au 1/5 en (p/V) dans l'eau. Les extraits hydroéthanoliques sont dilués au 1/2 (volume/volume) dans l'eau, filtrés à 0,45 µm puis dérivés. La calibration est réalisée en externe avec le kit de dosage. Les résultats obtenus sont présentés dans le tableau 1 ci-dessous.

**Tableau 1 : Teneurs en acides aminés libres**

| Solvant | FGE | EtOH/eau | EtOH/eau |
|---|---|---|---|
| Partie utilisée | Fleur | Fleur | graine |
| Teneur en acides aminés libres (en mg/100g) | 89,0 | 76,7 | 7,2 |

### Conclusion :

L'extrait fleur FGE est significativement plus riche en acides aminés libres que l'extrait fleur EtOH/eau. La teneur très faible en acides aminés libres de l'extrait graine EtOH/eau, prouve que la composition chimique des fleurs et des graines est significativement différente. Les extraits de fleur et les extraits de graine d'*Aesculus hippocastanum* ont manifestement une composition différente.

### Exemple 3: Recherche de la présence de saponosides et coumarines dans les extraits de fleurs d'Aesculus hippocastanum

La recherche de la présence de composés de type coumarines dans l'extrait FGE et EtOH/Eau de fleur d'*Aesculus hippocastanum* obtenus à l'exemple 1 est réalisée par HPLC-UV-DAD, en utilisant une colonne SymmetryShield 3,5 µm (150x4.6 mm) et des solutions de molécules étalons d'esculétine, d'esculine, de fraxine et de fraxétine. L'interprétation est réalisée par comparaison des temps de rétention et des spectres d'absorption UV des molécules étalons et des molécules présentes dans l'extrait.

La recherche de la présence de composés de type saponosides dans l'extrait FGE et EtOH/Eau de fleur d'*Aesculus hippocastanum* obtenus à l'exemple 1 est réalisée par HPLC-UV-DAD, en utilisant une colonne SymmetryShield 5 µm (250x4,6 mm) et une solution de molécule étalon d'aescine. L'interprétation est réalisée par comparaison des temps de rétention et des spectres d'absorption UV de la molécule étalon et des molécules présentes dans l'extrait.

**Tableau 2 : Résultat de la recherche de la présence de coumarines et de saponosides dans l'extrait de fleur d'Aesculus hippocastanum.**

| Solvant | FGE | EtOH/eau |
|---|---|---|
| Partie utilisée | Fleur | Fleur |
| Saponosides | absence | absence |
| Coumarines | absence | absence |

### Conclusion :

Contrairement à des extraits de graine ou d'écorce d'*Aesculus hippocastanum,* l'extrait fleur FGE ne contient pas de composés de types saponosides et coumarines pas davantage que l'extrait fleur EtOH/eau. Cette expérience montre que la composition chimique de l'extrait d'*Aesculus hippocastanum* ne dépend pas que du choix du solvant d'extraction mais aussi de la partie du végétal utilisée.

### Exemple 4: Effet d'un extrait fleur FGE d'Aesculus hippocastanum selon l'invention sur l'expression protéique du collagène IV dans les fibroblastes dermiques humains normaux.

### Protocole :

Un test fluoroimmunoassay a été réalisé et consiste à révéler l'antigène d'intérêt, en l'espèce le collagène IV. Cette méthode est semi-quantitative, hautement sensible et reproductible, et présente l'avantage de détecter la protéine d'intérêt sous sa forme native dans son environnement, sans procédé de dénaturation. Les fibroblastes, issus d'une biopsie de peau abdominale de donneurs âgés entre 30 et 60 ans, sont extraits et fixés au fond des puits à une densité de 8000 cellules par puits, et croissent pendant 96 heures dans un milieu défini. Les cellules sont ensuite cultivées pendant 48 heures avec l'extrait fleur FGE obtenu à l'exemple 1 testé à différentes concentrations ou avec le solvant d'extraction seul ou sans l'extrait dit contrôle. Les cellules sont alors lavées en tampon phosphate salin (PBS) avant d'être fixées, perméabilisées et saturées à la BSA (albumine sérique bovine) à 1% pendant 30 minutes. Les cellules sont incubées avec l'anticorps primaire (anti collagène IV) pendant 1 heure, lavées en tampon PBS, puis incubées avec l'anticorps secondaire lié au fluorochrome Eu-N1 pendant 1 heure. La fluorescence est lue sur un spectrofluorimètre (Tecan M1000) avec les filtres adéquats. Les mesures de fluorescence sont rapportées à la quantité d'ADN dosée.

Les résultats sont rassemblés dans le tableau 3 ci-dessous. L'expérience est effectués 6 fois (n=6). Les valeurs du tableau représentent les valeurs en pourcentage rapportées aux cellules contrôles non traitées. Les valeurs représentent la moyenne de plusieurs expériences (n=6) sur différents lots d'extraits. « Moy » désigne la moyenne et « EC » désigne l'écart type.

**Tableau 3 : Pourcentage d'expression protéique du collagène IV dans les fibroblastes dermiques humains normaux en fonction de la dose d'extrait fleur FGE d'Aesculus hippocastanum utilisée.**

| | Moy | EC |
|---|---|---|
| Contrôle | 100 | 10 |
| Solvant d'extraction à 0,5% (FGE) | 101,8 | 11,3 |
| Extrait d'*Aesculus hippocastanum* à 0,05% | 100,52 | 12,3 |
| Extrait d'*Aesculus hippocastanum* à 0,1% | 120,8 | 7,4 |
| Extrait d'*Aesculus hippocastanum* à 0,18% | 147,49 | 9,7 |
| Extrait d'*Aesculus hippocastanum* à 0,25% | 162,68 | 12,1 |
| Extrait d'*Aesculus hippocastanum* à 0,5% | 329,05 | 34,8 |

### Conclusion :

L'extrait fleur FGE obtenu selon l'invention induit une augmentation significative et dose-dépendante de l'expression protéique du collagène IV. Le solvant d'extraction seul n'induit pas d'augmentation d'expression du collagène IV.

L'extrait fleur FGE obtenu selon l'invention induit donc une amélioration de la synthèse protéique du réseau macromoléculaire de la jonction dermo-épidermique.

### Exemple 5: Effet d'un extrait fleur FGE, d'un extrait fleur EtOH/eau et d'un extrait graine FGE d'Aesculus hippocastanum sur l'expression protéique du collagène IV dans les fibroblastes dermiques humains normaux.

Le protocole est identique à celui de l'exemple 4 si ce n'est qu'on teste comparativement un extrait fleur EtOH/eau et de graine FGE obtenus à l'exemple 1 à une concentration de 0.25%.

Les résultats figurent dans le tableau suivant :

**Tableau 4 : Pourcentage d'expression protéique du collagène IV dans les fibroblastes dermiques humains normaux entre extraits fleur FGE, fleur EtOH/eau, graine FGE d'Aesculus hippocastanum.**

| | Moy | EC |
|---|---|---|
| Contrôle | 100 | 4.96 |
| Solvant d'extraction EtOH/Eau à 0,25% | 93.87 | 8.39 |
| Solvant d'extraction FGE à 0,25% | 93.96 | 13.56 |
| Extrait fleur EtOH/eau à 0,25% | 212.84 | 17.88 |
| Extrait fleur FGE à 0.25% (invention) | 411.72 | 28.23 |
| Extrait graine FGE à 0.25% | 102.21 | 12.76 |

L'extrait fleur FGE obtenu selon l'invention induit une augmentation environ 2 fois supérieure, de l'expression protéique du collagène IV comparativement au même extrait obtenu avec un solvant EtOH/Eau. L'extrait graine FGE n'induit pas d'augmentation d'expression du collagène IV.

### Exemple 6: Effet d'un extrait fleur FGE d'Aesculus hippocastanum selon l'invention sur l'expression protéique du collagène VII dans les fibroblastes humains normaux.

### Protocole :

La technique est la même que dans l'exemple 4 sauf que l'antigène d'intérêt est le collagène VII. Les résultats sont rassemblés dans le tableau 4 ci-dessous. L'expérience est effectués 6 fois (n=6). Les valeurs du tableau représentent les valeurs en pourcentage rapportées aux cellules contrôles non traitées. Les valeurs représentent la moyenne de plusieurs expériences (n=6) sur différents lots d'extraits. « Moy » désigne la moyenne et « EC » désigne l'écart type.

**Tableau 5 : Pourcentage d'expression protéique du collagène VII dans les fibroblastes 20 dermiques humains normaux en fonction de la dose d'extrait fleur FGE d'Aesculus hippocastanum utilisée.**

| | Moy | EC |
|---|---|---|
| Contrôle | 100 | 15,9 |
| Solvant d'extraction FGE à 0,5% | 93,6 | 6 |
| Extrait d'*Aesculus hippocastanum* à 0,05% | 110,2 | 6,4 |
| Extrait d'*Aesculus hippocastanum* à 0,1% | 130,1 | 13 |
| Extrait d'*Aesculus hippocastanum* à 0,18% | 159,5 | 13,2 |
| Extrait d'*Aesculus hippocastanum* à 0,25% | 144,7 | 12,5 |
| Extrait d'*Aesculus hippocastanum* à 0,5% | 151,5 | 16 |

### Conclusion :

L'extrait fleur FGE obtenu selon l'invention a induit une augmentation significative et dose dépendante de l'expression protéique du collagène VII. Le solvant d'extraction seul n'induit pas d'augmentation d'expression du collagène VII. L'extrait fleur FGE obtenu selon l'invention induit donc une amélioration de la synthèse protéique du réseau macromoléculaire de la jonction dermo-épidermique.

### Exemple 7 : Effet d'un extrait fleur FGE, d'un extrait fleur EtOH/eau et d'un extrait graine FGE d'Aesculus hippocastanum sur l'expression protéique du collagène VII dans les fibroblastes dermiques humains normaux.

Le protocole est identique à celui de l'exemple 6 si ce n'est qu'on teste comparativement un extrait fleur EtOH/eau et de graine FGE obtenus à l'exemple 1 à une concentration de 0.25%.

Les résultats figurent dans le tableau suivant :

**Tableau 6 : Pourcentage d'expression protéique du collagène VII dans les fibroblastes dermiques humains normaux entre extraits fleur FGE, fleur EtOH/eau, graine FGE d'Aesculus hippocastanum.**

| | Moy | EC |
|---|---|---|
| Contrôle | 100 | 4.96 |
| Solvant d'extraction EtOH/Eau à 0,25% | 100.11 | 12.99 |
| Solvant d'extraction FGE à 0,25% | 103.82 | 13.15 |
| Extrait fleur EtOH/eau à 0,25% | 146.01 | 11.90 |
| Extrait fleur FGE à 0.25% (invention) | 170.59 | 18.48 |
| Extrait graine FGE à 0.25% | 113.18 | 14.92 |

L'extrait fleur FGE obtenu selon l'invention induit une augmentation supérieure de l'expression protéique du collagène VII comparativement au même extrait obtenu avec un solvant EtOH/eau. L'extrait de graine mettant en œuvre le solvant FGE n'induit pas d'augmentation d'expression du collagène VII.

### Exemple 8: Effet d'un extrait fleur FGE d'Aesculus hippocastanum selon l'invention sur l'expression protéique de la fibronectine dans les fibroblastes dermiques humains normaux

### Protocole :

La technique est la même que dans l'exemple 4 mais avec les modifications suivantes :
- l'antigène d'intérêt est la fibronectine ;
- les cellules croissent pendant 24 heures dans un milieu défini avant d'être cultivées pendant 72 heures avec l'extrait fleur FGE obtenu à l'exemple 1 ;
- l'anticorps secondaire est lié au fluorochrome Alexa 488 ;
- La fluorescence est lue sur un système d'imagerie à haute résolution (INCELL AnalyzerTM1000) et les mesures de fluorescence sont quantifiées par analyse d'images (aire de fluorescence/nombre de noyaux colorés par le Hoechst). Les résultats sont rassemblés dans le tableau 5 ci-dessous.

L'expérience est effectués 3 fois (n=3). Les valeurs du tableau représentent les valeurs en pourcentage rapportées aux cellules contrôles non traitées. Les valeurs représentent la moyenne de plusieurs expériences (n=3) sur différents lots d'extraits. « Moy » désigne la moyenne et « EC » désigne l'écart type.

**Tableau 7 : Pourcentage d'expression protéique de la fibronectine dans les fibroblastes dermiques humains normaux traités avec l'extrait fleur FGE d'Aesculus hippocastanum à 0,5%.**

| | Moy | EC |
|---|---|---|
| Contrôle | 100 | 3,7 |
| Extrait fleur FGE d'*Aesculus* hippocastanum à 0,5% | 117,3 | 2,7 |

### Conclusion :

L'extrait fleur FGE obtenu selon l'invention a induit une augmentation significative de l'expression protéique de la fibronectine dans des fibroblastes dermiques humains normaux. L'extrait fleur FGE obtenu selon l'invention induit donc une amélioration de la synthèse protéique de la matrice du derme.

### Exemple 9 : Effet d'un extrait fleur FGE et d'un extrait graine FGE d'Aesculus hippocastanum sur l'expression protéique de la fibronectine dans les fibroblastes dermiques humains normaux

Le protocole est identique à celui de l'exemple 8 si ce n'est qu'on teste comparativement un extrait fleur FGE et de graine FGE obtenus à l'exemple 1 à une concentration de 0.25%.

Les résultats figurent dans le tableau suivant :

**Tableau 8 : Pourcentage d'expression protéique de la fibronectine dans les fibroblastes dermiques humains normaux traités avec l'extrait de fleur FGE ou de graine FGE d'Aesculus hippocastanum**

| | Moy | EC |
|---|---|---|
| Contrôle | 100 | 6.64 |
| Solvant d'extraction FGE à 0,25% | 102.07 | 4.39 |
| Extrait fleur FGE à 0.25% (invention) | 120.73 | 4.14 |
| Extrait graine FGE à 0.25% | 98.33 | 5.20 |

L'extrait fleur FGE obtenu selon l'invention induit une augmentation significative de l'expression protéique de la fibronectine dans des fibroblastes dermiques humains normaux alors que l'extrait de graine FGE n'induit pas d'augmentation d'expression de la fibronectine.

### Exemple 10: Effet d'un extrait fleur FGE d'Aesculus hippocastanum selon l'invention sur l'expression protéique du collagène IV dans les kératinocytes humains normaux.

### Protocole :

La technique est la même que dans l'exemple 4 sauf qu'elle est mise en œuvre sur des kératinocytes humains normaux et que l'antigène d'intérêt est le collagène IV. Les résultats sont rassemblés dans le tableau 6 ci-dessous. L'expérience est effectués 6 fois (n=6). Les valeurs du tableau représentent les valeurs en pourcentage rapportées aux cellules contrôles non traitées. Les valeurs représentent la moyenne de plusieurs expériences (n=6) sur différents lots d'extraits. « Moy » désigne la moyenne et « EC » désigne l'écart type.

**Tableau 9 : Pourcentage d'expression protéique du collagène IV dans les kératinocytes 10 humains normaux en fonction de la dose d'extrait fleur FGE d'Aesculus hippocastanum utilisée.**

| | Moy | EC |
|---|---|---|
| Contrôle | 100 | 12 |
| Solvant d'extraction FGE à 0,5% | 84 | 4,5 |
| Extrait d'*Aesculus hippocastanum* à 0,05% | 117,7 | 5 |
| Extrait d'*Aesculus hippocastanum* à 0,1% | 158 | 19,7 |
| Extrait d'*Aesculus hippocastanum* à 0,18% | 173,9 | 11,9 |
| Extrait d'*Aesculus hippocastanum* à 0,25% | 257,4 | 26 |
| Extrait d'*Aesculus hippocastanum* à 0,5% | 349,2 | 53,3 |

### Conclusion :

L'extrait fleur FGE obtenu selon l'invention a induit une augmentation significative et dose dépendante de l'expression protéique du collagène IV. Le solvant d'extraction seul n'induit pas d'augmentation d'expression du collagène IV. L'extrait fleur FGE obtenu selon l'invention induit donc une amélioration de la synthèse protéique du réseau macromoléculaire de la jonction dermo-épidermique.

### Exemple 11 : Effet d'un extrait fleur FGE et d'un extrait graine FGE d'Aesculus hippocastanum sur l'expression protéique du collagène IV dans les kératinocytes humains normaux.

Le protocole est identique à celui de l'exemple 10 si ce n'est qu'on teste comparativement un extrait de fleur FGE et de graine FGE obtenus à l'exemple 1 à une concentration de 0.25%.

Les résultats figurent dans le tableau suivant :

**Tableau 10 : Pourcentage d'expression protéique du collagène IV dans les kératinocytes humains normaux traités avec l'extrait de fleur FGE ou de graine FGE d'Aesculus hippocastanum**

| | Moy | EC |
|---|---|---|
| Contrôle | 100 | 8.15 |
| Solvant d'extraction FGE à 0,25% | 99.52 | 12.40 |
| Extrait fleur FGE à 0.25% (invention) | 142.20 | 13.20 |
| Extrait graine FGE à 0.25% | 102.97 | 9.95 |

L'extrait fleur FGE obtenu selon l'invention induit une augmentation significative de l'expression protéique du collagène IV dans les kératinocytes humains normaux alors que l'extrait de graine FGE n'induit pas d'augmentation d'expression du collagène IV.

### Exemples 12 : compositions comprenant l'extrait d'Aesculus hippocastanum selon l'invention.

**Crème de jour**

| Nom des ingrédients | Composition (% massique) |
|---|---|
| EAU DÉMINÉRALISÉE | QS100 |
| CARBOPOL ULTREZ 21 | 0,10 |
| GLYCÉRINE | 3,00 |
| BUTYLÈNE GLYCOL | 5,00 |
| PENTYLÈNE GLYCOL | 2,00 |
| GOMME DE XANTHANE | 0,20 |
| EMULIUM® DELTA | 3,00 |
| PHÉNOXYÉTHANOL | 0,30 |
| HUILE DE SILICONE | 4,00 |
| EXTRAIT DE L'INVENTION | 2,00 |
| HYDROXIDE DE SODIUM (solution aqueuse 10%) | 0,25 |
| Total | 100,00 |

**Gel crème naturel hydratant**

| Nom des ingrédients | Composition (% massique) |
|---|---|
| EMULIUM® MELLIFERA | 2,5 |
| SQUALANE NATUREL | 2,0 |
| DICAPRYLYL CARBONATE | 15,0 |
| EAU DÉMINÉRALISÉE | QS100 |
| GLYCÉRINE | 20,0 |
| AMIDON DE TAPIOCA | 5,0 |
| EXTRAIT DE L'INVENTION | 2,0 |
| ÉTHYLHEXYLGLYCÉRINE | 0,1 |
| PHÉNOXYÉTHANOL | 0,9 |
| PARFUM | 0,2 |
| Total | 100,0 |

**Spray nourrissant**

| Nom des ingrédients | Composition (% massique) |
|---|---|
| EMULIUM® MELLIFERA | 2,00 |
| LABRAFAC™ CC | 5,00 |
| LIPOCIRE™ A | 3,00 |
| MYRISTATE D'OCTYLDODECYL | 5,00 |
| HUILE DE SILICONE | 2,00 |
| EAU DÉMINÉRALISÉE | QS100 |
| STEAROYLE GLUTAMATE DE SODIUM | 0,50 |
| GLYCÉRINE | 5,00 |
| BUTYLÈNE GLYCOL | 5,00 |
| PEMULEN TR-2 | 0,10 |
| GOMME DE XANTHANE | 0,15 |
| EXTRAIT DE L'INVENTION | 2,00 |
| TEA STEARATE | 0,10 |
| PHÉNOXYÉTHANOL | 0,45 |
| ÉTHYLHEXYLGLYCÉRINE | 0,05 |
| PARFUM | 0,05 |
| Total | 100,00 |

**Emulsion eau dans huile**

| Nom des ingrédients | Composition (% massique) |
|---|---|
| PLUROL® DIISOSTEARIQUE CG | 4,0 |
| HUILE DE RICIN HYDROGÉNÉE | 2,0 |
| COMPRITOL® 888 CG | 1,0 |
| SQUALANE NATUREL | 3,0 |
| ALKANE NATUREL | 22,0 |
| SILICATE DE MAGNESIUM | 3,0 |
| EAU DÉMINÉRALISÉE | QS100 |
| GLYCÉRINE | 3,0 |
| SULFATE DE MAGNESIUM 7H2O CODEX | 1,5 |
| CHLORURE DE SODIUM CODEX | 1,5 |
| PARFUM | 0,3 |
| EXTRAIT DE L'INVENTION | 2,0 |
| PHÉNOXYÉTHANOL | 0,9 |
| ÉTHYLHEXYLGLYCÉRINE | 0,1 |
| Total | 100,0 |

## Revendications

1. Extrait d'*Aesculus hippocastanum* **caractérisé en ce qu'**il présente :
- une teneur en flavonoïdes comprise entre 10 et 200 mg/100g d'extrait ; et/ou
- une teneur en acides aminés libres comprise entre 10 et 200 mg/100g d'extrait,
et **en ce qu'**il est susceptible d'être obtenu par un procédé d'extraction mettant en œuvre un solvant d'extraction consistant en un mélange de fructose et de glycérine comprenant éventuellement de l'eau, ledit procédé d'extraction comprenant une étape d'extraction solide/liquide de la fleur, suivie d'une seconde étape de séparation solide /liquide et enfin d'une troisième étape de récupération de la phase liquide.

2. Extrait selon la revendication 1, **caractérisé en ce que** le solvant consiste en un mélange de fructose, de glycérine et d'eau dans des proportions molaires comprises entre 1:1:3 et 1:1:7, préférentiellement dans des proportions molaires d'environ 1:1:5.

3. Extrait selon l'une des revendications précédentes, **caractérisé en ce que** la fleur est sèche.

4. Extrait selon la revendication 3 , **caractérisé en ce que** la partie de la plante est la fleur sèche et broyée.

5. Extrait selon l'une des revendications précédentes, **caractérisé en ce qu'**il présente une teneur en flavonoïdes comprise entre 50 et 100 mg/100g d'extrait.

6. Extrait selon l'une des revendications précédentes, **caractérisé en ce qu'**il présente une teneur en acides aminés libres comprise entre 50 et 100 mg/100g d'extrait.

7. Utilisation non thérapeutique de l'extrait selon l'une des revendications 1 à 6 ou de compositions le comprenant, pour le traitement cosmétique de la peau et/ou des muqueuses, en particulier pour l'amélioration de l'aspect de la peau et/ou des muqueuses, pour l'amélioration de la résistance et/ou de l'élasticité cutanée, pour le traitement ou la prévention des signes du vieillissement de la peau, des rides, ou du relâchement cutané.

8. Utilisation non thérapeutique de l'extrait selon l'une des revendications 1 à 6 ou de compositions le comprenant pour stimuler la synthèse du collagène IV, du collagène VII et de la fibronectine.

9. Composition cosmétique comprenant l'extrait selon l'une des revendications 1 à 6.

10. Composition selon la revendication 9, **caractérisée en ce que** l'extrait représente entre 0,1 % et 10 %, de préférence entre 0,5 % et 5 % en poids de la composition.

## Patentansprüche

1. Rosskastanienextrakt, **dadurch gekennzeichnet, dass** er aufweist:
- einen Flavonoid-Gehalt zwischen 10 und 200 mg/100g Extrakt; und/oder
- einen Gehalt an freien Aminosäuren zwischen 10 und 200 mg/100g Extrakt;
und dadurch, dass er durch ein Extraktionsverfahren hergestellt wird, bei dem ein Extraktionslösungsmittel, bestehend aus einer Mischung aus Fructose und Glyzerin, eventuell außerdem noch aus Wasser, eingesetzt wird, wobei dieses Extraktionsverfahren einen Extraktionsschritt Feststoff/Flüssigkeit der Blüte, gefolgt von einem zweiten Schritt der Abscheidung Feststoff/Flüssigkeit und schließlich einen dritten Schritt des Auffangens der flüssigen Phase enthält.

2. Extrakt nach Anspruch 1, **dadurch gekennzeichnet, dass** das Lösungsmittel aus einer Mischung aus Fructose, Glyzerin und Wasser, in einem Molverhältnis zwischen :1:3 und 1:1:7, vorzugsweise in einem Molverhältnis von ungefähr 1:1:5, besteht.

3. Extrakt nach einem der vorangehenden Ansprüche, **dadurch gekennzeichnet, dass** es sich um getrocknete Blüten handelt.

4. Extrakt nach Anspruch 3, **dadurch gekennzeichnet, dass** es sich bei dem Pflanzenteil um die getrocknete und zerkleinerte Blüte handelt.

5. Extrakt nach einem der vorangehenden Ansprüche, **dadurch gekennzeichnet, dass** er einen Flavonoidgehalt zwischen 50 und 100 mg/100g Extrakt aufweist.

6. Extrakt nach einem der vorangehenden Ansprüche, **dadurch gekennzeichnet, dass** er einen freien Aminosäuregehalt zwischen 50 und 100 mg/100g Extrakt aufweist.

7. Nicht-therapeutischer Einsatz des Extraktes nach einem der Ansprüche 1 bis 6 oder von Zusammensetzungen, in denen er enthalten ist, zur kosmetischen Behandlung der Haut und/oder der Schleimhäute, insbesondere zur Verbesserung des Aussehens von Haut und/oder Schleimhäuten, zur Verbesserung der Festigkeit und/oder der Hautelastizität zur Behandlung oder Vorbeugung der Anzeichen für Hautalterung, Falten oder Hauterschlaffung.

8. Nicht-therapeutischer Einsatz des Extraktes nach einem der Ansprüche 1 bis 6 oder von Zusammensetzungen, in denen er enthalten ist, zur Stimulierung der Collagen IV-, Collagen VII- und der Fibronektin-Synthese

9. Kosmetische Zusammensetzung, die den Extrakt nach einem der Ansprüche 1 bis 6 enthält.

10. Zusammensetzung nach Anspruch 9, **dadurch gekennzeichnet, dass** der Extrakt zwischen 0,1 % und 10 %, vorzugsweise zwischen 0,5 % und 5 % des Gewichtes der Zusammensetzung bildet.

## Claims

1. Extract of *Aesculus hippocastanum* **characterized in that** it has:
- a flavonoid content of between 10 and 200 mg/100g of extract; and/or
- a free amino acid content of between 10 and 200 mg/100g of extract,
and that is is capable of being obtained by an extraction process comprising an extraction solvent consisting of a mixture of fructose and glycerin optionally comprising water, said extraction process comprising a step of solid/liquid extraction of the flower, followed by a second step of solid/liquid separation and finally of a third step of recovery of the liquid phase.

2. Extract according to claim 1, **characterized in that** the solvent consists of a mixture of fructose, glycerin and water in molar proportions between 1:1:3 and 1:1:7, preferably in proportions molars of approximately 1:1:5.

3. Extract according to one of the preceding claims, **characterized in that** the flower is dry.

4. Extract according to claim 3, **characterized in that** the part of the plant is the dry and ground flower.

5. Extract according to one of the preceding claims, **characterized in that** it has a flavonoid content of between 50 and 100 mg/100g of extract.

6. Extract according to one of the preceding claims, **characterized in that** it has a free amino acid content of between 50 and 100 mg/100g of extract.

7. Non-therapeutic use of the extract according to one of claims 1 to 6 or of compositions comprising it, for the cosmetic treatment of the skin and/or mucous membranes, in particular for improving the appearance of the skin and/or mucous membranes, for improving the resistance and/or skin elasticity, for the treatment or prevention of aging of the skin, wrinkles, or sagging skin.

8. Non-therapeutic use of the extract according to one of claims 1 to 6 or of compositions comprising it to stimulate the synthesis of collagen IV, collagen VII and fibronectin.

9. Cosmetic composition comprising the extract according to one of claims 1 to 6.

10. Composition according to Claim 9, **characterized in that** the extract represents between 0.1% and 10%, preferably between 0.5% and 5% by weight of the composition.
